Europäisches Patentamt

(19) European Patent Office

Office européen des brevets

(11) Publication number: 0 137 691
A1

(12) EUROPEAN PATENT APPLICATION

(21) Application number: 84305859.5

(22) Date of filing: 28.08.84

(51) Int. Cl.⁴: C 12 P 21/02
C 07 K 13/00
//C12N15/00

(30) Priority: 29.08.83 US 527209

(43) Date of publication of application:
17.04.85 Bulletin 85/16

(84) Designated Contracting States:
AT BE CH DE FR GB IT LI LU NL SE

(71) Applicant: MELOY LABORATORIES, INC.
6715 Electronic Drive
Springfield Virginia 22151(US)

(72) Inventor: Braude, Irwin Allen
5711 Walnut Wood Lane
Burke Virginia(US)

(74) Representative: Laredo, Jack Joseph et al,
Elkington and Fife High Holborn House 52/54 High
Holborn
London, WC1V 6SH(GB)

(54) Production of immune interferon and its mRNA.

(57) Process for the production of IFN-γ comprising incubating for a period of time at least sufficient for the production of IFN-γ, a viable cell suspension which includes (a) leukocytes, (b) an inducer for production of IFN-γ, and (c) a modulator for said inducer selected from the group consisting of mezerein and 12, 13-phorbol butyrate, said inducer and said modulator being present in said suspension in amount sufficient to stimulate production of IFN-γ; process for producing substantially homogeneous IFN-γ; process for producing IFN-γ mRNA; and cloning process for production of IFN-γ. Products disclosed are substantially homogeneous IFN-γ and its 2 species; and the host cells containing the recombinant cloning vehicle containing DNA segment coding for IFN-γ.

## PRODUCTION OF IMMUNE
## INTERFERON AND ITS mRNA

This invention relates to processes for producing immune interferon and its mRNA, and to homogeneous immune interferon ("IFN-$\gamma$").

More particularly, one aspect of the invention relates to a process for the production of high yields if human IFN-$\gamma$ from induced human leukocytes modulated with mezerein or 12,13-phorbol dibutyrate. Preferably, the leukocytes are induced by a calcium ionophore selected from A-23187, ionomycin, and 1,1-dimethyl-6,6,7,7,8,8,8-heptafluoro-3,5-octanedione.

Another aspect of this invention relates to IFN-$\gamma$ which has been purified to substantial homogeneity.

A further aspect of this invention relates to a process for the recovery of IFN-$\gamma$ mRNA, and to a cloning process for the production of IFN-$\gamma$ .

Interferon was discovered by Isaacs and Lindenmann in 1957, who observed that fluids from virus-infected cell cultures contained a protein which could react with normal cells to render them resistant to infection by a wide variety of viruses. In addition to potent antiviral effects,

interferon possesses anticellular, immunoregulatory and antitumor activities. The use of interferon in the treatment of cancer and viral infections in animals has met with some successes, and consequently its use in man is of great interest.

Interferons are classified into three major species designated alpha or leukocyte interferon (IFN-$\alpha$), beta or fibroblast interferon (IFN-$\beta$) and gamma or immune interferon (IFN-$\gamma$). IFN-$\alpha$ and IFN-$\beta$ are induced by viruses or synthetic polynucleotides.

IFN-$\gamma$ has been induced in lymphocytes by specific antigens or by certain T-cell mitogens. It is believed that protein mitogens cause cell membrane receptors to form clusters which are termed "patches". Presumably, the patching event allows a rapid influx of calcium into the cell which, for a reason not entirely understood, initiates production of IFN-$\gamma$.

IFN-$\gamma$ may be distinguished from the IFN-$\alpha$ and IFN-$\beta$ species of interferon by its inability to be neutralized by antibodies directed against either IFN-$\alpha$ or IFN-$\beta$, and its ability to be neutralized by antibodies directed against IFN-$\gamma$. IFN-$\gamma$ may also be characterized by the fact that its biological activity is largely destroyed by being placed in a low pH environment, such as pH 2, and also when brought into contact with sodium dodecyl sulfate.

Interferon related research has primarily concentrated on the IFN-$\alpha$ and IFN-$\beta$ species. For example, considerable work on producing IFN-$\alpha$ has been done by Dr. Kari Cantell in Finland. See, "Production and Preparation of Human Leukocyte Interferon", K.E. Morgensen and D. Cantell, 1 Pharmac. Ther. C., 369-381, 1977. The amino acid sequences of IFN-α and IFN-β have recently been identified by protein sequence analyses and work on the sequences of their respective cloned complementary DNA. Furthermore, there is now evidence that multiple sub-species of IFN-α and IFN-β exist.

Very little is known about the structure of IFN-γ or its biological properties. It is now believed, however, that IFN-γ may be more active as a cell growth inhibitory agent, immunoregulatory agent and antitumore agent, and it may possess the ability to potentiate both the antiviral and anticellular effects of either IFN-α or IFN-β. Thus, IFN-γ may be greater interest than IFN-α and IFN-β.

A variety of T-cell mitogenic proteins have been identified for inducing IFN-γ from unprimed lymphocytes. Some of the more commonly used proteins are plant lectins such as concanavalin A and phytohemagglutinin A, or other proteins known to be mitogenic such as staphylococcal enterotoxin A and staphylococcal protein A.

An article, "Human Immune Interferon: Induction in Lymphoid Cells by a Calcium Ionophore," by F. Dianzani et al, 29 Interferon and Immunity, 561-563, August 1980, described the use of the calcium ionophore A-23187 as an IFN-γ inducer. (A-23187 is described in U.S. Patent No. 3,932,823.) Calcium ionophore A-32187 is not a protein. It is believed that A-32187, due to its lipophilic properties and high affinity for calcium, permits a rapid influx of calcium across the cell membrane thereby simulating the patching mechanism of the protein inducers. However, using the Dianzani et al. procedure, the IFN-γ activity is barely perceptible; only 10-100 units per ml. of IFN-γ activity has been observed. As used herein, a unit is equivalent to 50% inhibition of viral cytopathic effects. (Comparison of titers among different laboratories is not readily made because there is no accepted international standard for IFN-$\gamma$ .)

This invention teaches a process for producing high yields of IFN-$\gamma$ and its mRNA which is reliable, efficient and functional. It also discloses genetic engineering techniques for producing clones which can express IFN-$\gamma$ .

The present invention relates to a process for the production of IFN-$\gamma$ comprising incubating for a period of time at least sufficient for the production of IFN-$\gamma$ , a viable cell suspension which includes leukocytes, an inducer for production of IFN-$\gamma$ , and a modulator for said inducer selected from mezerein or 12,13-phorbol butyrate, said inducer and said modulator being present in the suspension in amounts sufficient to stimulate production of IFN-$\gamma$ , separating the supernatatant from the cell suspension and purifying the IFN-$\gamma$ by

(a) adsorbing the IFN-$\gamma$ onto a column containing Controlled Pore Glass beads and eluting with ammonium sulfate;

(b) adsorbing the IFN-$\gamma$ containing eluate of step (a) onto a column containing Concanavalin A-Sepharose, lentil lectin Sepharose or pea lectin-agarose and eluting with a buffer containing a sugar;

(c) adsorbing the IFN-$\gamma$ containing eluate of step (b) onto a column containing Heparin-Sepharose or Procian Red-agarose and eluting with a buffer containing a high salt content; and

(d)   passing the IFN-γ containing eluate of step (c) through a gel-filtration column equilibrated in high salt and recovering purified IFN-γ;

The improvement comprising dialyzing the partially purified interferon against a chemically compatible buffer and contacting the solution of dialyzed partially pure interferon with a column of cationic exchanger resin for a period of time sufficient to accomplish equilibrated adsorption of the interferon to the exchanger in the presence of a buffer at pH about 9.0 to 10.0, washing the resin having interferon adsorbed thereon from the soluble fraction with a buffered solution until the optical density of the eluate at 280 nm is about 0, eluting the adsorbed interferon with a desorbing buffer of about 10-30 mM tris-HCl at pH of about 9-10 containing 50 mM NaCl; the step being performed prior to step (d) and after steps (a), (b), (c).

The leukocytes may originate from a variety of sources including human, monkey, sheep, cow, pig and chicken. Particularly, the viable cell suspension includes leukocytes, a modulator selected from the group consisting of mezerein or 12,13-phorbol dibutyrate, and an IFN-γ inducer. Preferably, the IFN-γ inducer is a calcium ionophore selected from the group consisting of A-23187, ionomycin, or 2,2-dimethyl-6,6,7,7,8,8,8-heptafluoro-3,5-octanedione (hereinafter "FOD").

At the end of the incubation period, both IFN-γ and IFN-γ mRNA may be extracted from the cell suspension. The IFN-γ is found in the supernatant and may be purified to homogeneity by adsorbing and eluting the crude IFN-γ through a series of columns. A technique for purifying IFN-γ is disclosed in my U.S. Patent No. 4,482,026 which is incorporated herein by reference.

0137691

The IFN-ɣ mRNA is contained in the cells. It is extracted from the cells by a process which generally involves first removing the large DNA strands and proteins from the lysed cells, and then separating the mRNA from other types of RNA and small strands of DNA is a poly (U) column. The mRNA that contains high IFN-ɣ activity is selected by size fractionation.

This mRNA is now useful for expressing IFN-ɣ in clones. The principle advantage of cloning is that by placing the DNA which codes for IFN-ɣ into a host cell, such as bacteria or yeast, there is a potential for harvesting enormous amounts of IFN-ɣ. Briefly, this is accomplished by adding the isolated mRNA to a viral enzyme, reverse transcriptase, which reads the mRNA code and transcribes a single-stranded complementary DNA. Double-stranded DNA is achieved in the presence of DNA polyerase or reverse transcriptase and is placed into a host cell, such as the bacteria E. coli, by means of genetic engineering recombinant techniques such as described in U.S. Patent No. 4,237,224. For example, insertion of the double-stranded DNA into the host cell is accomplished by first selecting a cloning vehicle, such as a plasmid, which is compatible with the host cell and carries a phenotypical trait, such as penicillin resistance. Enzymes which are capable of cleaving specific cites on the cloning vehicle remove a portion of the vehicle approximately the size of the DNA coding for IFN-ɣ to be inserted. The DNA and the cloning vehicle are joined together with DNA ligase. The recombinant cloning vehicle is then introduced into the host in the presence of calcium chloride by the process of transformation. Afterwards the cells that have taken up the recombinant cloning vehicle molecule, the transformants, are selected, usually by use of antibiotics, and thereafter selectively isolated by their ability to hybridize with mRNA from IFN-γ induced cells. These transformants are then screened for expression of IFN-γ. The IFN-γ ds-DNA from selected transformants are placed in optimal expressing vehicles for large-scale production of IFN-γ.

The calcium ionophores, A-23187 and ionomycin, are antibiotics produced by microorganisms. The calcium ionophore FOD is synthetic material, and it has been shown that its biological effects resemble those described for A-23187. The main difference between FOD and A-32187, for purposes of this invention, is that greater concentrations of FOD appear to be necessary to produce similar yields of IFN-γ. However, there may prove to be no significant distinction in optimal concentrations of A-32187 and FOD upon discovery of a solvent for FOD which is capable of reducing FOD to its monomeric (non-micellar) form and is not cytotoxic. Practically, the use of greater concentrations of FOD is irrelevant since FOD is inexpensive and readily available in large quantities.

Mezerein, the modulator, is a poly-cyclic $C_{38}H_{38}O_{10}$ hydrocarbon with molecular weight of 654. It is an extract from the plant Daphne mezereun L. Mezerein has been shown to be as effective a lymphocyte mitogen as the potent tumor promoting agent, 12-0-tetradecanoylphorbol 13-acetate (TPA). Thus, its discovery as a potent modulator for IFN-γ inducers makes it extremely useful.

The alternative modulator useful in this invention, 12,13-phorbol dibutyrate, is a phorbol ester which, like mezerein, is a potent modulator for IFN-$\gamma$ inducers.

The combination of the modulator and the inducer produces significantly more titer of IFN-$\gamma$ than were they used individually, and more than would be expected if the effects of the two were merely additive.

It has also been demonstrated that IFN-$\gamma$ inducers, particularly one of the calcium ionophores, in combination with a modulator stimulates production of higher immune IFN-$\gamma$ titer than the unmodulated IFN-$\gamma$ inducers, staphylococcal enterotoxin A, phytohemagglutinin A and concanavalin A.

The combination of mezerein and calcium ionophore inducers, particularly A-32187, reliably and consistently produces high yields of IFN-$\gamma$. Additionally, by virtue of the relatively low molecular weights of calcium ionophore and mezerein, their hydrophobicity, and in the case of A-23187, its high affinity for certain divalent cations, they are easily removed from the IFN-$\gamma$ product. Thus, there is far less concern with toxicity of the mezerein/calcium ionophore system than with the other known inducers which are all proteins and are difficult to remove from other proteins found in the cell suspension. In addition, the inducer proteins may tend to co-purify with the IFN-$\gamma$ thereby contaminating the final product, making it impractical for clinical use. Thus, the mezerein/calcium ionophore system is particularly advantageous for production of human IFN-$\gamma$ for use in clinical trials, and also for recovery of the mRNA from the incubated cell suspension for future cloning.

The accompanying drawings will assist in understanding the invention disclosed herein. Figure 1 is a generalized flow chart of the various process steps. Figures 2 and 3 are dose curves associated with Example 4 herein.

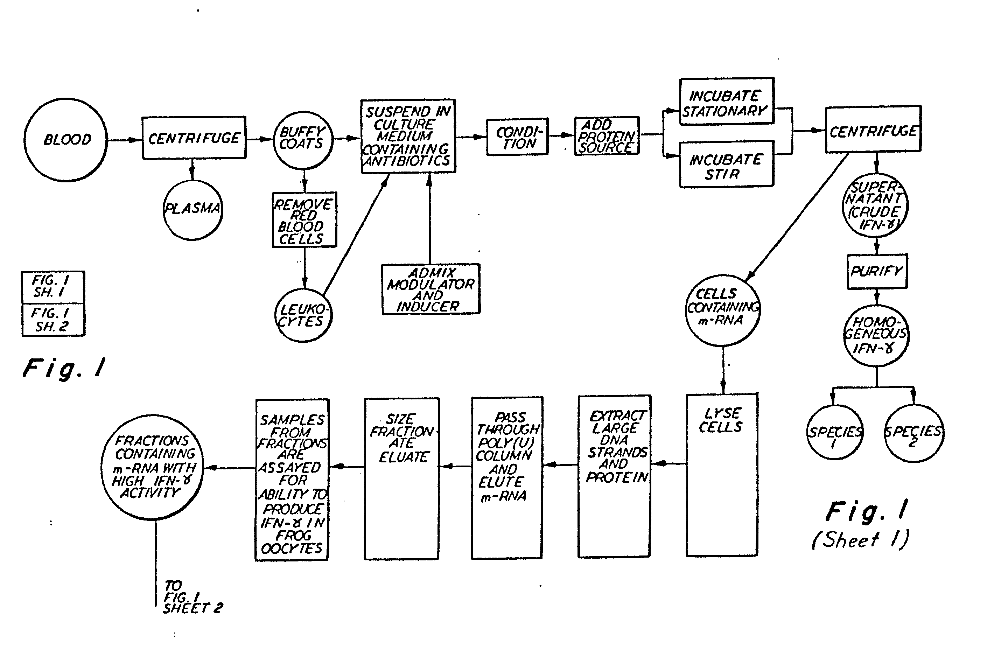

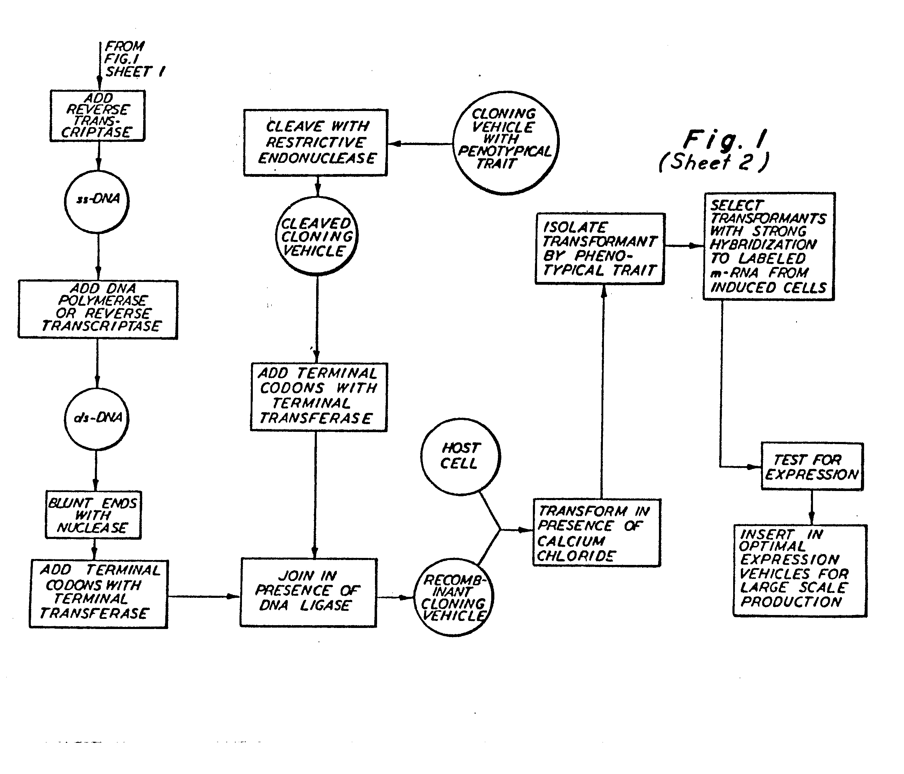

The Figure 1 flow chart is a diagrammatic aid for the specification and claims and does not fully disclose all the features of this invention which are described in this specification.

The flow chart describes the process for production of IFN-$\gamma$ and its purification to homogeneity; the process for production of mRNA useful for cloning IFN-$\gamma$; and the process for producing clones capable of expressing IFN-$\gamma$.

With reference to the Figure 1 flow chart, buffy coats are isolated from whole blood by centrifugation and then suspended in a culture medium containing antibiotics. Alternatively, the erythrocytes are removed from the buffy coats and the remaining leukocytes are suspended in the culture medium. The modulator and inducer are admixed with the suspension and conditioned for a short period. A nutrient sources is added and the cell suspension is incubated under either stationary or agitated conditions. The incubated cell suspension is then centrifuged to separate the supernatant from the cells. The supernatant contains the crude IFN-$\gamma$ which is purified to homogeneity. The cells contain the IFN-$\gamma$ mRNA which is used in the cloning process.

The cells are lysed and the IFN- $\gamma$ mRNA is extracted by a process comprising removing the large DNA strands and proteins, passing the remaining RNA through a poly (U) column to remove rRNA and small strands of DNA, eluting mRNA from the column and size fractionating the mRNA. Samples from the various sizing fraction are assayed for their ability to code for IFN- $\gamma$ by in vivo translation in frog oocytes. The fractions corresponding to the highest IFN- $\gamma$ activity are selected for cloning.

The ds-DNA to be inserted in the cloning vehicle is prepared from the extracted mRNA in two stages. First the ss-DNA complementary to the mRNA is synthesized in the presence of reverse transcriptase. This ss-DNA acts as the template to synthesize its complementary ss-DNA in the presence of reverse transcriptase or DNA polymerase, and through hydridization achieving the ds-DNA. The ds-DNA is made blunt ended and appropriate terminal codons are added. The ds-DNA is then joined with a cleaved cloning vehicle with appropriate terminal codons in the presence of DNA ligase. This recombinant cloning vehicle is placed in the host cell by the transformation process. The resulting transformants are isolated by means of the phenotypical trait carried by the cloning vehicle. The transformants are further selected by their ability for strong hybridization to labelled mRNA from IFN- $\gamma$ induced cells and optionally followed by positive hybridization selection. The candidate transformants are screened in a translational system, preferrably, Xenopus laevis oocytes. The IFN- $\gamma$ ds-DNA from these transformants may be removed and inserted into optimal expressing vehicles which may be grown for large-scale production of IFN- $\gamma$ .

0137691

In accordance with the preferred embodiments of this invention, buffy coats containing human leukocytes are provided as a viable cell source for human IFN-$\gamma$ production. Buffy coats may be obtained by a variety of standard clinical techniques, such as by leukoporesis or on a hemoetics machine.

One suitable method involves collecting whole blood (usually 100 ml.) by venipuncture from healthy donors in acid-citrate-dextrose solution or in heparinized containers. The acid-citrate-dextrose solution and heparin are normal additives to blood units preventing coagulation of the blood cells upon standing. The whole blood is centrifuged and the plasma fraction removed. The layer immediately beneath the serum of the centrifuged material is the buffy coat. The buffy coat consists primarily of leukocytes and some erythrocytes. One component of the leukocytes are T-cell lymphocytes. It is believed that these lymphocytes are responsible for production of IFN-$\gamma$.

Another method of isolating the leukocytes is by the Ficoll-Hypaque gradient method as described in A. Boyum, "Isolation of Leukocytes from Human Blood", 21 <u>Scand. J. Clin. Lab. Invest. Suppln.</u> 31-50 (1968). A substantial portion of the cells derived from this technique are lymphocytes, of which the T-cell originated lymphocytes are present. The remaining mononuclear leukocyte cells are $\beta$-cell lymphocytes, monocytes and null cells.

In U.S. Patent No. 4,376,821 a process was disclosed in which human peripheral blood was treated with a buffered ammonium chloride solution to lyse material. It has now been discovered that when the preferred inducers are used, yields of human IFN-$\gamma$ are improved up to five times if the buffy coat material is not pretreated with the ammonium chloride solution. In the preferred embodiment, buffy coat material is used directly in the modulation-induction procedure of this invention.

The buffy coat material is suspended in a culture medium, with about $1.0 \times 10^6$ to about $9.0$ to $10^6$ leukocyte cells/ml. of cell suspension as the concentration range. The optimum concentration range is from about $4.0 \times 10^6$ to about $7.0 \times 10^6$ cells/ml. The preferred concentration is $5.0 \times 10^6$ cells/ml. Commercially available culture media which have been found to be especially suitable are RPMI 1640 available from GIBCO laboratories, Grand Island, New York, and when using the preferred inducers, Dulbecco's Minimal Essential Media (DMEM) available from Meloy Laboratories, Inc. Springfield, Virginia.

In some cases, it is desirable to add antibiotics to the culture medium before suspending the buffy coats to prevent contaminating bacterial growth. Broad spectrum antibiotics such as 100 units of pennicilli per ml. culture medium, 100 ug. of streptomycein per ml. culture medium, or 100 ug. of gentamicin per ml. culture medium may be used. Although the inclusion of antibiotics in the culture medium inhibits bacterial growth, the antibiotics themselves are contaminants in the sense that they must be removed from the extracted interferon product. Even trace amounts of antibiotics, if not completely removed from the interferon product, may cause allergic reactions when administered to susceptible patients.

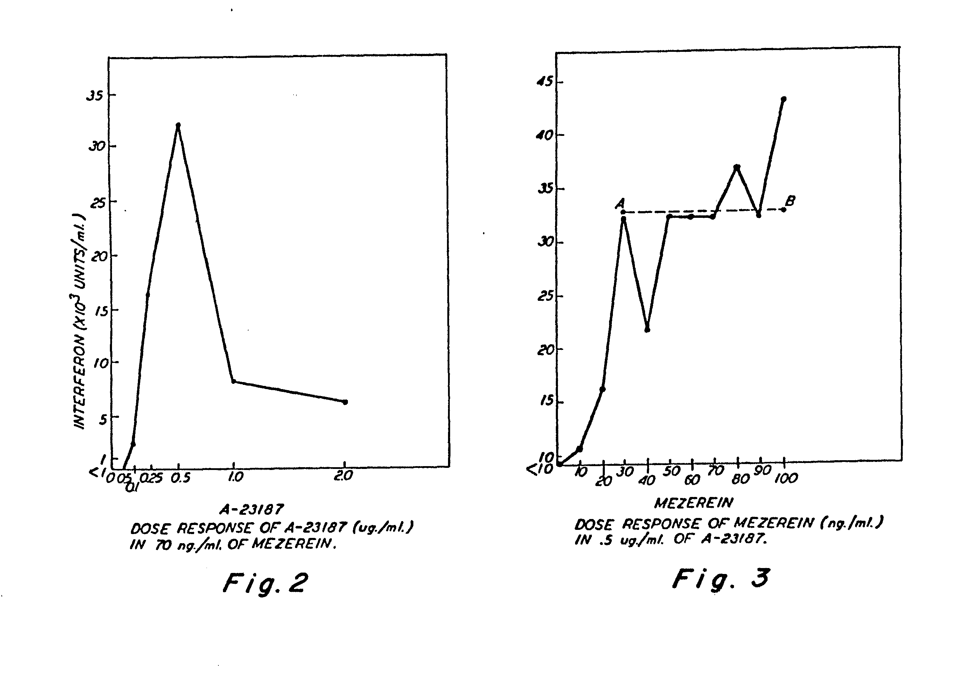

A modulator selected from the group consisting of mezerein and 12,13-phorbol dibutyrate is added to the culture medium containing the suspended buffy coat material. These modulators are commercially available from CCR, Inc., Eden Prairie, Minnesota. The modulator may be added in a minimum amount of about 0.7 ng./ml. of cell suspension. The upper limit does not appear to be critical because at optimal concentrations of modulator the yield of IFN-$\gamma$ plateaus. There may be a divergence in the optimal concentration of modulator among different preparations or batches; this is likely due to the level of purity of the modulator in each batch. The purer the modulator, the less that is necessary to produce maximum yields of IFN-$\gamma$ . The optimal amount of modulator to be used for a particular batch of modulator is preferably determined by constructing a calibration grid. (See, e.g., Example 4, and Figure 3,) This may be done by plotting a series of dose responses for varying concentrations of mezerein as a function of varying concentrations of the inducer. The optimal proportion of modulator to be included in the cell suspension is in the range of the first point of the plateau of the dose response curve (e.g., dotted line segment A-B of Figure 3). Based on the calibration grids constructed for two separate batches of mezerein, the minimum concentration for mezerein to induce optimal quantities of IFN-$\gamma$ varies between 7 ng./ml. and 30 ng./ml. Although it has been found that the concentration of mezerein required for optimal modulation of A-32187 may vary from batch to batch, neither the kinetics of IFN-$\gamma$ production nor the shape of the A-32187 dose response curve is altered.

U.S. Patent No. 4,376,821 described introduction of calcium ionophore A-23187 and a modulator into the culture medium that contains peripheral blood leukocytes. The amount of A-23187 per ml. of cell suspension was in the range of between about 0.1-10.0 ug./ml. and preferably 0.10-2.0 ug./ml. Thereafter, the mixture was incubated for between about 12 hours and 7 days at about 37°C. while gently stirring the culture (spin culture) at a rate of about 10 rpm. The spin culture suspension was maintained under a blanket of air/$CO_2$, which can be maintained by feeding $CO_2$ into the suspension through micron pore size air stones to distribute the gas. Thus a uniform $CO_2$ mixture of about 5% was maintained at all times.

It has now been found that A-23187 may very effectively be used within the preferred proportional range of 0.15-0.8 µg./ml. of cell suspension under stationary culture conditions during the incubation period. Preferably, A-23187 in the amount of between 0.25-0.6 ug./ml. is used. Within this range, stationary cultures have occasionally yielded 2-3 times higher titers of IFN-$\gamma$ than when the cultures are agitated.

The incubation temperature should remain at about body temperature, i.e., 37°C. The temperature may be varied to the extent the cells remain viable, and within the range of viability, there appears to be a relationship between temperature and incubation time: higher temperatures appear to require shorter incubation times. Induction of IFN-$\gamma$ has been observed after about 12 hours of incubation, and induction lasts until the cells are no longer viable, up to as long as about 12 days. Three to 4 days is the preferred incubation period on the basis of maximal yield per unit of time.

It has been demonstrated that there is an initial phase of production between about 20 hours and 40 hours. THis is followed by a loss of activity and then a second phase of production.

The greatly improved yield of IFN-$\gamma$ resulting from the stationary culture incubation and the smaller preferred proportion of A-23187 is thought to be due to the availability of direct cell contact of adjacent cells throughout the stationary incubation period, rather than continual random cell contact which occurs when the cell suspension is agitated during the incubation period. In any event, increased yields of up to 3 times have been occasionally noted.

In addition to increased yields, the stationary culture incubation process also permits considerably smaller batches to be made that yield the same proportion of IFN-$\gamma$ as when the larger batch is used in the spinner culture incubation process. As a consequence, there is a significant reduction in the amount of contaminants that co-reside with the IFN-$\gamma$. Moreover, there is a significant cost savings by using less A-32187. Thus, without reducing the overall yield, the efficiency of the earlier process is materially improved.

A-23187 alone will induce cells to produce barely detectable amounts of human IFN-$\gamma$. By a mechanism which is not entirely understood, the addition of a modulator appears to increase the number of cells capable of responding to A-23187, to increase the number of cells capable of responding to A-23187, and thus greatly enhancing the yield of IFN-$\gamma$. For instance, in reproduction of the Dianzani et al. article (calcium ionophore A-23187, was employed without a modulator), only 10-100 units of antiviral activity per ml. was measured. In contrast, the combination of mezerein with an inducer has consistently induced cells to increased yields of IFN-$\gamma$. Particularly, mezerein in combination with A-23187 may produce IFN-$\gamma$ in the range of up to 400,000 and more units of anti-viral activity per ml. In specific experiments yields of up to 80,000 and more units per ml have been obtained.

An alternative calcium ionophore which is a practical and an effective inducer is 2,2-dimethyl-6,6,7,7,8,8,8-heptafluoro-3,5-octanedione (FOD). It is commercially available from the Aldrich Chemical Company, catalog #17,516-1. FOD has been demonstrated to closely resemble calcium ionophore A-23187 in its effects on biological systems. ("A Synthetic Ionophore For Ca$^{2+}$: Studies With Model and Biological Systems", Gomperts et al., 117 Eur. J. Biochem., pp. 559-562, 1981).

Ionomycin is also a potent IFN-$\gamma$ inducer. It is a calcium ionophore available from Squibb, Inc.

Experiments using FOD as an inducer for IFN-$\gamma$ production ranging from 30-300 ug./ml. of cell suspension have been conducted. While FOD has been added at concentrations 100-1,000 times greater than A-23187 it is believed that the greater FOD concentration requirement may be due to its being in a micellar state. Use of a solvent for FOD which is capable of reducing it to its monomeric (non-micellar) form and which is not cytotoxic will eliminate this variance in concentrations between the two calcium ionophores. In the presence of such a solvent the minimum concentration proportion for FOD might be as low as .10 μg./ml. of cell suspension. As a practical matter the high concentrations of FOD are not significant because it is inexpensive and readily available in large quantities.

It should be noted that the order in which the modulator and calcium ionophore inducer are included in the cell suspension is not controlling. They may be added in any order, or simultaneously. However, when the inducer is a protein it should be added at about 2 hours after the modulator.

It has also been found that IFN-$\gamma$ yield is further increased if a nutrient source such as a protein source, for example, fetal calf serum or calf serum is added to the cell suspension in an amount sufficient to keep the cells alive. That amount is generally about 3% to about 15% at final concentration and, preferably from about 3% to 10% (volume/volume) serum/cell suspension. It is preferable to add the nutrient source after the cell suspension has been conditioned for about 2-6 hours at about body temperature.

It is essential to the invention that the cells remain viable during incubation. Thus, the appropriate amount of cell suspension to be included in a culture container is determined by the capacity of that particular container such that a suitable volume of air/$CO_2$ makes contact with the cell suspension to maintain viability over the incubation period. Conditions must be such that a dynamic exchange of $CO_2$ and other volatile metabolites of the cell suspension can be established between the suspension media and the air volume. Suitably, relatively shallow apparatus, with liquid depths of such as approximately 3mm. in deep trays or 5-10 mm. in tissue culture tubes are used to carry the stationary culture during incubation, thereby providing a large surface area-to-volume ratio.

It is desirable to initiate the incubation period with a pH such that the cells will remain viable. For example, the culture may be initially buffered to a pH of about 7.0-7.9, preferably about 7.2-7.4.

After the cell culture suspension has incubated for a period of time sufficient to produce desired yields of crude IFN-$\gamma$, the cells are separated from the supernatant, for example, by centrifugation, at 1000-3000 X g, depending on the culture volume, for the necessary time, e.g., 10-60 minutes, at 4°C. The yields of crude IFN-$\gamma$ that result from the crude cell suspensions may contain up to 400,000 units and more IFN-$\gamma$ activity per ml. cell suspension. The residual cells can be used for reinducing IFN-$\gamma$ production, or for recovery of IFN-$\gamma$ mRNA, or both. The supernatant containing the crude IFN-$\gamma$ may be stored at 4°C.

A partial purification process for crude IFN-$\gamma$ is described in, "Partial Purification and Characterization of Human (immune) Interferon", Yip et al., 78 Proc. Natl. Acad. Sci. U.S.A. No. 3, 1601-05, (1981).

A technique for purifying IFN-$\gamma$ is disclosed in Serial No. 293,775. Generally, the process concerns passing the crude IFN-$\gamma$ through 4 columns. One column contains Controlled Pore Glass beads. The crude IFN-$\gamma$ is adsorbed onto the column and eluted with ammonium sulfate. A second column contains either Concanavalin A-Sepharose, lentil

0137691

lectin-Sepharose or pea-lectin agarose. The IFN-$\gamma$ is adsorbed onto this column and then eluted with a buffer containing sugar. A third column contains Heparin-Sepharose or Procian Red-agarose. The IFN-$\gamma$ is adsorbed onto this column and eluted with a salt buffer. The final column is a gel-filtration column equilibrated in high salt.

More specifically, the crude IFN-$\gamma$ is contacted with Controlled Pore Glass beads in a chromatographic column for a period of time sufficient to accomplish equilibrated adsorption of the crude IFN-$\gamma$ to the beads in the presence of a neutral pH buffer such as phosphate buffered saline (PBS) at about pH 7.2. If the crude IFN-$\gamma$ is produced from cell suspensions containing red blood cells, then before contacting the crude IFN-$\gamma$ to the CPG beads tris (hydroxymethyl) amino methane is added to bring the concentration to 500 mM and buffered to a pH of 9.5 to 9.7. The glass beads are then washed with a chemically compatible buffer such as an aqueous solution containing tris (hydroxymethyl) amino methane at a concentration of 500 mM, and buffered to about pH 9.5, to remove the unbound contaminant until the optical density of the eluate at 280 nm. is about 0. The glass beads having IFN-$\gamma$ adsorbed thereon are next washed with a neutral pH buffer solution until the optical density at 280 nm. is about 0, and then adsorbed IFN-$\gamma$ is eluted from the glass beads with an aqueous solution of 2M ammonium sulfate at pH about 9.0. Optionally, the above pH adjustment step may be applied to the crude IFN-$\gamma$ regardless of whether or not red cells are present.

This eluate is then passed through a column packed with an adsorbent selected from the group consisting of Concanavalin A-Sepharose, lentil lectin-Sepharose and pea lectin-agarsoe for the period of time sufficient to accomplish equilibrated adsorption of the IFN-$\gamma$ to the adsorbent in the presence of a neutral pH buffer such as phosphate buffered saline. The adsorbent having IFN-$\gamma$ adsorbed thereon is washed with ammonium sulfate until the optical density of the eluate at 280 nm. is about 0. The adsorbent is then washed with a phosphate buffered saline until the optical density of the eluate at 280 nm. is about 0. Elution of the adsorbed IFN-$\gamma$ is then carried out with a phosphate buffered saline containing from 0.1 M to 2.0 M alpha-methyl D-mannoside or 1-methyl D-glucoside.

The IFN-$\gamma$ eluate is then contacted with Heparin-Sepharose or Procian Red-agarose and equilibrated in a neutral pH buffered solution. The IFN-$\gamma$ is washed with a neutral pH buffered solution such as PBS containing about 1 M alpha-methyl D-mannoside or 1-methyl D-glucoside to remove the unbound material until the optical density of the eluate at 280 nm. is about 0. Again, the adsorbent is washed with a neutral pH buffered solution until the optical density is about 0 to remove some of the bound material. The adsorbed IFN-$\gamma$ is eluted with a second neutral pH buffered solution containing a high concentration of salt.

The IFN-$\gamma$ may be adsorbed and eluted in these three columns in any order.

The IFN-$\gamma$ may be further purified by subjecting the elution fraction to gel-filtration treatment equilibrated in a neutral pH buffered solution containing a high concentration of salt, and then eluting with the same equilibrating buffer. The solutes within the elution fractions may be separated according to their molecular weights.

Analysis of the resulting eluate material from the gel-filtration column that is passed through SDS-PAGE demonstrated 4 bands. These are at approximately 20,000 daltons, 25,000 daltons, 47,000 daltons, 63,000 daltons. The molecular weight measurements are subject to an accuracy of about ± 3,000 daltons. An amino acid analyses on each band suggested that the 25,000-63,000 dalton bands have essentially the same amino acid contents, and it is thereupon concluded that the 47,000 and 63,000 bands represent multiples, i.e., dimers and trimers of the 25,000 dalton band. The 25,000-63,000 dalton bands represent the homogeneous species IFN-$\gamma_1$. Biological activity assays indicated that the 25,000 and 47,000 dalton bands possess IFN-$\gamma$ activity; the 63,000 dalton band IFN-$\gamma$ will possess biological activity, but this has not yet been confirmed.

The 20,000 dalton band represents the homogeneous species IFN-$\gamma_2$ since its amino acid analysis suggests it is different from that of the 25,000-63,000 dalton bands. The 20,000 dalton band IFN-$\gamma$ will possess biological activity, but this has not yet been confirmed.

Alternatively, the IFN-$\gamma$ may be purified as described in commonly assigned U.S. application Serial No. 437,660, filed October 29, 1982, of the same inventive entity incorporated herein by reference. The procedure is outlined in Example 9 below.

The homogeneous IFN-$\gamma$ prepared by either method is particularly useful for physiochemical characterization structure studies, antibody production, and clinical application.

After incubating the cell suspension for up to 3 days, the mRNA may be extracted from the cells. It is believed that after 3 days enzymes start digesting the mRNA. First, the cells are lysed and the large contaminants removed. For example, the cells are isolated by centrifugation and then lysed with sodium acetate and SDS or in a guanidine thiocyanate solution. Large strands of DNA and proteins are removed with phenol extraction or by centrifugation in cesium chloride in a cellulose nitrate tube. The remaining mRNA pellet is precipitated in ethanol and then resuspended in a salt buffer. To further remove DNA and rRNA the suspension is bound to a column containing poly (U) sepharose and washed with a buffer. The mRNA is eluted with formamide and size fractionated on sucrose gradients. Samples from the fractions are injected in cells capable of translating IFN- $\gamma$ mRNA, such as <u>Xenopus laevis</u> oocytes. After the oocytes have had sufficient time to produce the IFN- $\gamma$ , the IFN- $\gamma$ activity is measured. The fractions corresponding to the highest IFN- $\gamma$ relative to RNA are selected for cloning.

The fractions containing high IFN- $\gamma$ activity are then added to reverse transcriptase. This enzyme reads the mRNA code and transcribes its single-stranded complementary DNA. Reverse transcriptase or DNA polymerase is added to achieve the double-stranded DNA. Alternatively, the isolation of IFN- $\gamma$ mRNA may be effected as provided in Example 8 below.

This double-stranded DNA may be placed into a host cell, such as the bacteria <u>E. coli</u>, by relying on general genetic-engineering/recombinant DNA techniques such as described in the summary.

The preferred embodiments of this invention are further illustrated by the examples which follow.

EXAMPLE 1

Production of Crude IFN- $\gamma$ ; Removed Erythrocytes; Spin
Cultures

The red blood cells contained in buffy coats were lysed with 7 volumes of 0.83% (weight/volume) buffered ammonium chloride and immediately centrifuged at approximately 25 X g for 20 minutes to separate the lysed red blood cells from the buffy coat containing the leukocytes. The leukocytes were suspended at about 5.0 X $10^6$ cells per ml. in DMEM medium (Meloy).

Mezerein, the modulator, at a final concentration of about 7 ng./ml. of cell suspension and the calcium ionophore A-23187, at a final concentration of about 2.0 ug./ml. of cell suspension were admixed with the leukocytes and medium to form the cell suspension. The cell suspension was conditioned for 2 hours at 37°C. The fetal calf serum was then added until the fetal calf serum in the culture reached 10%.

The culture was incubated for 72 hours at 37°C. in a gently stirred container. After incubation the culture (crude IFN- $\gamma$ ) was harvested by centrifugation at 2620 X g at 4°C. for 20 minutes. The supernatant containing crude IFN- $\gamma$ was separated from the cells and particulate material.

EXAMPLE 2

Dose Response for A-32187

IFN-$\gamma$ production from human leukocytes as a function of varying A-23187 concentrations modulated by mezerein at a concentration of 7 ng./ml. is shown in the dose response data shown in Table I. The mezerein was prepared from a single batch received from CCR, Inc. The IFN-$\gamma$ was prepared under stationary incubation conditions (See Example 4).

TABLE I

| A-23187 Added (ug./ml.) | IFN-$\gamma$ Titer (Units/ml. |
| --- | --- |
| 0.01 | 30 |
| 0.05 | 30 |
| 0.10 | 30 |
| 0.15 | 1200 |
| 0.20 | 360 |
| 0.25 | 720 |
| 0.50 | 720 |
| 1.0 | 120 |
| 10.0 | 30 |

The optimum yield of IFN-$\gamma$ was between about 0.15 to 1.0 ug./ml. of A-23187 for this particular batch of mezerein.

Table I titers of IFN-$\gamma$ were measured in a semi-micro c.p.e.-inhibition assay using WISH cells as the indicator cells and Vesicular Stomatitis Virus (VSV) as the challenge virus. Significantly higher titers of IFN-$\gamma$ were measured when the challenge virus encephalomyocarditis virus (EMC) was used. (See Example 5.) EMC is more sensitive to the anti-viral effects of IFN-$\gamma$ .

EXAMPLE 3

Modulated A-32187 Verses Unmodulated Protein Inducers

The human IFN-$\gamma$ modulated by mezerein and induced by A-23187, as in Example 1, was compared to IFN-$\gamma$ production induced by optimum concentration of staphylococcal enterotoxin A (SEA), phytohemagglutinin-P (PHA-P) and concanavalin A (Con A), without a modulator. Leukocyte cultures (5 X $10^6$ cells/ml. DMEM medium), SEA, PHA-P and Con A. Modulated A-23187 induced 2-4, 5-10 and 10-20 times, respectively, more IFN-$\gamma$ than the unmodulated proteins.

Virtually all of the detectable interferon of Examples 1, 2 and 3 were IFN-$\gamma$. Treatment at pH 2 resulted in 99% loss of activity in 1 hour, whereas control human IFN-$\alpha$ was only slightly affected. Antibodies to IFN-$\alpha$ had little effect IFN-$\gamma$, whereas such antibodies completely neutralized the antiviral effect of IFN-$\alpha$. Furthermore, the IFN-$\gamma$ was not active on bovine cells, and it demonstrated a different chromatographic profile when compared to human IFN-$\alpha$.

## EXAMPLE 4

Production of Crude IFN-$\gamma$ ; Buffy Coats; Stationary Culture

Whole blood, approximately 100 ml. volumes, was collected by venipuncture from healthy human donors in acid citrate-dextrose solution. The whole blood was centrifuged and the plasma faction removed. The buffy coat material was suspended at about 5.0 X $10^6$ leukocyte cells per ml. in DMEM medium (Meloy).

Stock solutions of A-23187 and mezerein were prepared in DMSO at concentrations of 50 mg./ml. and 1mg./ml., respectively. Working solutions were then prepared in DMEM. Mezerein, at a final concentration of at about 70 ng./ml. of culture, and A-23187, at a final concentration of 0.5 ug./ml., were added to the leukocyte and medium to form the cell suspension. The mezerein used in this Example was from a different batch than the mezerein used in Example 1, 2 and 3. (Upon constructing calibration grids using mezerein in 10 ng./ml. increments ranging from 10 ng./ml. to 100 ng./ml. and A-23187 in varying concentrations ranging from .05 ug./ml. to 2 ug./ml. it was determined that 30 ng./ml. of cell suspension was the optimal concentration of mezerein for this batch. 30 ng./ml. corresponds to the first point of the plateau (e.g., dotted-line segment A-B of Figure 3) of the dose response curve. 70 ug./ml. was used merely as a safety margin.)

A dose response curve of A-23187 in 70 ng./ml. of mezerein is shown in Figure 2.

A dose response curve for mezerein in .5 ug./ml. of A-23187 is shown in Figure 3. The plateau is determined by the average of the dose response points beginning with the point corresponding to the first significant production of IFN-$\gamma$. In this case that point was approximately 32 X $10^3$ times of IFN-$\gamma$ activity.

The cultures were then poured into tissue culture tubes in which the depth of the culture was approximately 10 mm. and were then conditioned for 2 hours at 37°C. Fetal calf serum was then added until the fetal calf serum in the cell suspension reached 10%. The cell suspension was then incubated for 3 days at 37°C. under stationary conditions and under a air/$CO_2$ blanket containing 5% $CO_2$. At the end of the incubation period the cultures (crude IFN-$\gamma$) were harvested by centrifugation at 1000 X g at 4°C. for 60 minutes. The supernatant containing crude IFN-$\gamma$ was separated from the cells and particulate material.

EXAMPLE 5

Yield of Crude IFN-$\gamma$

A sample of the IFN-$\gamma$ of Example 4 was titrated in a semi-micro c.p.e.-inhibition assay using WISH cells as the indicator cells and encephalomyocarditis virus (EMC) as the challenge virus. IFN-$\gamma$ was approximately $30 \times 10^3 - 40 \times 10^3$ units/ml.

Virtually all of the detectable interferon in this system was IFN-$\gamma$. Treatment at pH 2 resulted in 99% loss of activity in 1 hour, whereas control human IFN-$\alpha$ was only slightly affected. Antibodies to IFN-$\alpha$ had little effect on IFN-$\gamma$, whereas such antibodies completely neutralized the antiviral effect of IFN-$\alpha$. Furthermore, the IFN-$\gamma$ was not active on bovine cells, and it demonstrated a different chromatographic profile when compared to human IFN-$\alpha$.

Using the method of Example 4 and EMC as the challenge virus, IFN-$\gamma$ titers may range from about 8000 units/ml. to about 400,000 units/ml. and more; and have ranged from about 8,000 units/ml to about 80,000 units/ml and more.

## EXAMPLE 6

Purification to Homogeneity

The following solutions were prepared: solution of 500 mM tris (hydroxymethyl) amino methane at pH 9.6, then add 12N HCl to bring pH to 9.5, referred to as tris; solution of 2M ammonium sulfate at pH 9.0 (w/NH$_4$OH); neutral pH buffered solution of phosphate buffered saline (PBS) at pH 7.2 having a 1M solution of alpha-methyl D-mannoside; and phosphate buffered saline solution (PBS) at pH 7.2 containing 2M sodium chloride.

The following columns were prepared: 2.6 cm. X 30 cm. chromatographic column was loaded with Controlled Pore Glass beads (CPG) (about 160 ml. bed volume); 1.6 cm. X 16 cm. chromatographic column was loaded with Concanavalin A-Sepharose (Con A) (about 32 ml. bed volume); and, a 1.6 cm. X 8 cm. chromatographic column was loaded with Heparin-Sepharose (H/S) (about 16 ml. bed volume). These three columns were equilibrated with phosphate buffered saline. A solution of the centrifuged crude IFN-$\gamma$ was adjusted to 500 mM with powdered tris, the pH adjusted to 9.5 with 12N HCl and recentrifuged. The adjusted crude IFN-$\gamma$ was loaded onto the CPG column at a flow rate of about 500 ml./hr. (about 94 ml./cm.$^2$/hr.). The column was monitored with a UV monitor at 2.0 AU and the unbound material collected into a beaker. The CPG column was washed with a 500 mM tris solution, pH 9.5, until the optical density (OD$_{280}$) was 0. The column was monitored at 2.0 AU and the unbound material collected. The column was washed with PBS until OD$_{280}$ returned to the baseline. (Flow rate 80 ml./hr. overnight). The eluted material was collected into a second beaker. The CPG column, was eluted with 2M ammonium sulfate at pH 9.0 at a flow rate of 24 ml./hr., and 10 ml. fractions were collected. When the second peak began to appear on the monitor, the inlet of the Con A column was connected into the outlet of the monitor and the outlet of the Con A column into the fraction collector. The CPG column eluted onto the Con A column at a flow rate of 24 ml./hr. (12 ml./cm.$^2$/hr. through Con A). The column was monitored as above until the $OD_{280}$ reached baseline.

The CPG column was disconnected from the monitor and the monitor connected to the Con A column. The Con A column was washed with PBS until $OD_{280}$ returned to the baseline. The flow rates and collections were monitored as above. The inlet of the H/S column was connected to the outlet of the monitor and the outlet of the H/S to the fraction collector. The Con A column was eluted with the PBS sugar solution. The collection was monitored as above until the $OD_{280}$ returned to baseline.

The Con A column was disconnected from the monitor and the H/S connected to the monitor. The H/S column was washed with PBS until $OD_{280}$ returned to the baseline. The flow rate and collection were monitored as above. The H/S column was eluted with 2M NaCl in PBS, pH 7.2 until $OD_{280}$ returned to the baseline. The flow rate was monitored as above and 3 ml. fractions were collected. The H/S peak fractions were selected by $OD_{280}$, and then pooled.

The pooled material was loaded onto the drained bed of the first P-100 column at 20 ml./hr. by gravity (about 80% bed height pressure head). After the sample was loaded, the sides of column were washed with 2M NaCl in PBS, pH 7.2. The flow was stopped and the column topped off with 2M NaCl PBS. The flow was then opened and the column was run at 24 ml./hr. in PBS, pH 7.2 with 2M NaCl. The system was monitored at 0.5 AU and 10 ml. fractions were collected.

EXAMPLE 7

Yield of Pure IFN-γ

The results in Table II show the degree of purification and recovery of human IFN-γ when purified by the sequential purification techniques described above.

TABLE II

| Description | Titer ($\log_{10}$ units/ml.) | Volume (ml.) | Recovery (%) | Protein (mg./ml.) | Specific Activity ($\log_{10}$ units/mg) |
|---|---|---|---|---|---|
| Crude | 3.7 | 3600 | NA | 18.46 | 2.4 |
| P-100 Column: | | | | | |
| Fraction 123 | 6.2 | 9.5 | 84 | 0.020 | 7.9 |
| Fraction 124 | 6.2 | 9.5 | 84 | 0.016 | 8.0 |

Based on SDS-PAGE separation procedures when purified IFN-γ preparation is segmented into 4 bands. The bands have a molecular weight 20,000 daltons, 25,000 daltons, 47,000 daltons and 63,000 daltons, ± 3,000 daltons. A sample of purified IFN-γ prepared by a process such as in Example 6 was passed through a denturing gel system to separate the IFN-γ into bands; the bands were then cut out and confirmed for homogeneity. These bands were subject to an amino acid analysis.

The amino acid analysis was run on a 20,000 dalton band, two 25,000 dalton bands (one band had a molecular weight of 25,000 daltons and the other had a molecular weight of 28,000 daltons), a 45,000 dalton band and a 66,000 dalton band. The individual protein bands were hydrolyzed into their separate amino acids. Each band was then run through a Durham Amino Acid Analyzer which separated the amino acids and quantitively measured the amino acid residues per protein. Comparison of the ratio of the relative peak heights between amino acids for the 25,000, 28,000, 45,000 and 66,000 dalton bands were sufficiently similar to conclude that these bands represent the same protein, and that the 45,000 dalton band is a dimer and the 66,000 dalton band is a trimer of the 25,000 dalton band.

Comparison of the ratio of the relative peak heights between amino acids for the dalton band and the 25,000 dalton band were sufficiently different to conclude the 20,000 dalton band represents a different species of IFN- $\gamma$ .

The purified IFN- $\gamma$ is shown to consist of two homogeneous species, namely IFN- $\gamma_1$ (MW about 25,000 daltons and its dimer and trimer), and IFN- $\gamma_2$ (MW about 20,000 daltons).

The following Table III shows the number of amino acid residues per IFN-$\gamma$ protein molecule. This Table is based on the results of a run through the amino acid analyzer for each band.

TABLE III

| Amino Acids | 25,000 Daltons | 28,000 Daltons | 45,000 Daltons* | 66,000 Daltons |
|---|---|---|---|---|
| Aspartic Acid | 23.5 | 29.0 | 77 | 55 |
| Threonine | 17.7 | 9.7 | | 32 |
| Serine | 30.7 | 29.5 | | 68 |
| Glutamic Acid | 36.0 | 29.7 | 83 | 93 |
| Proline | 5.4 | 7.7 | - | 21 |
| Glycine | 38.3 | 42.4 | 127 | 106 |
| Alanine | 24.3 | 17.2 | 71 | 64 |
| Valine | 10.6 | 8.4 | - | 27. |
| Isoleucine | 2.8 | 5.2 | - | 18 |
| Leucine | 12.9 | 19.4 | 26 | 38 |
| Phenylalanine | 5.3 | 16.0 | 21 | 15 |
| Histidine | 2.2 | - | 5 | 10 |
| Lysine | 9.6 | 15.6 | 30 | 30 |
| Arginine | 7.6 | 23.3 | - | 19 |
| Methionine | - | - | - | 5 |
| Cysteine | - | - | - | - |
| Tyrosine | - | - | - | - |
| Tryptophan | - | - | - | - |

---

\* The high numbers for the 45,000 dalton bands were caused by an elevated baseline.

\*\* This represents the total number of residues for Threonine and Serine.

0137691

## EXAMPLE 8

### Isolation of IFN-$\gamma$ mRNA

The cells, which remained after the supernatant containing the crude IFN-$\gamma$ of Example 4 was separated, were used in this Example.

The cells were centrifuged in a Ficoll Hypaque density gradient to isolate viable lymphocytes. The lymphocytes were lysed by dissolving them in 5-10 times their volume in a solution of 4.2 M guanidine thiocyanate, 25 mM sodium citrate, pH 7.0, 0.1 M 2-mercaptoethanol, 0.5% sarkosyl and 0.1% antifoam A. The solution was then homogenized on a sonic blender for one minute to reduce the viscosity by breaking up DNA strands. Twenty ml. of the solution was layered over 15 ml. of 5.7 M cesium chloride in 0.01 M EDTA in a SW27 cellulose nitrate centrifuge tube, and centrifuged for 72 hours at 22,000 rpm at 15°C. After aspirating the guanidinium thocyanate solution and decanting the cesium chloride solution, the remaining RNA pellet was rinsed in 500 1. of cold 70% ethanol to remove excess salt. The mRNA was dissolved in a solution of 0.01 M tris-HCl, pH 7.4 and 0.001 M EDTA, and then precipitated overnight by making the solution 70% ethanol. The ethanol was decanted and the remaining RNA pellet air dried and suspended in a salt buffer. The mRNA was bound to a column of poly (U) Sepharose 4B by passing the mRNA over the column in a solution containing 10 mM HEPES, pH 7.4, 3 mM EDTA, pH 7.4, 0.5% SDS and 300 mM NaCl. After rinsing the unbound RNA from the column, the poly-(A)-containing mRNA was eluted from the column in a solution containing 2.5 mM EDTA, pH 7.4, 1.0 mM HEPES, pH 7.4 and 70% deionized formamide maintained at

35

55-to-65°C. The mRNA was precipitated in 70% ethanol two times and dissolved in 0.01 M tris-HCl, pH 7.4 and 0.001 M EDTA. The mRNA was then size fractionated over a 5-29.9% isokinetic sucrose gradient in a SW41 rotor by centrifuging 27,000 rpm for 17 hours. Twenty-nine fractions were collected from the gradient and samples from selected fractions were assayed for their ability to produce IFN-$\gamma$ in Xenopus laevis oocytes.

IFN-$\gamma$ mRNA activity of selected fractions collected in Example 8, is shown in Table IV.

### TABLE IV

| Sample | Units of IFN-$\gamma$ per mg. mRNA | Units of IFN-$\gamma$ in Sample |
|---|---|---|
| Total mRNA[a] | 61 | 180.0 |
| Sucrose Gradients: | | |
| Fraction 11 | 2,560 | 3.0 |
| Fraction 12 | 240 | 3.1 |
| Fraction 13 | $\gg$2,560[b] | $\gg$3.3 |

[a] mRNA refers to material eluting from the poly (U) Sepharose 4B column.

[b] Protection from cytopathic effects of viral replication was provided at all dilutions of oocyte extract tested.

1 Construction and Selection of Transformant

Single-stranded cDNA was transcribed from the above #11 Fraction in the presence of avian myeloblastosis virus reverse transcriptase and a four-fold weight excess of oligo(dT) primer to mRNA. Double stranded cDNA was transcribed from a ss-cDNA template with reverse transcriptase. The ds-cDNA was made blunt-ended by incubation with $S_1$ nuclease. The reaction mixture consisted of 0.2 M sodium acetate pH 4.5, 0.4 M sodium chloride, 2.5 mM zinc sulfate and 1-5 units of $S_1$ nuclease per ng. of ds-cDNA in a final reaction volume of 100 μl. The ds-cDNA was incubated at 37°C. for 1 hr., extracted with phenol:chloroform, ethanol precipitated, and then subjected to size fractionation by electrophoresis on an 8% polyacrylamide gel.

Reaction products with a length of 300 base pairs or longer were eluted, dialyzed against $H_2O$ and lyophilized Terminal transferase was employed to add approximate 30 dCMP residues to the ds-cDNA 3'termini. About 15 residues of dCMP were added in a similar reaction to the plasmid pBR322 that had been previously cleaved by PstI restriction endonuclease. Equimolar amounts of dC-tailed ds-cDNA and dG-tailed plasmid were annelaed in 150 mM NaCl, 10 mM tris-HCl pH 7.6 and 1 mM EDTA at a final DNA concentration of 10 μg./ml. The annelaing mixture was placed at 75°C. and the temperature was lowered to 0°C. over a 5 hr. time period. Transformation of E. coli RR1 was carried out in the presence of calcium chloride. Transformants were selected by growth on tetracycline.

## Transformants Screened for IFN $\gamma$ cDNA Clones

Transformants were inoculated into individual wells of 96-well microtiter plates, grown to mid-log phase, adjusted to 8% dimethyl sulfoxide, and then maintained as frozen stocks at -70°C. Transformants were "screened" by growing the bacteria on replicate nitrocellulose filters using a 96-prong inoculating apparatus.

Filters were hybridized with $^{32}$P-labeled ss-cDNAs synthesized from mRNA isolated from normal and IFN-$\gamma$ induced human buffy coat cells. The filters were then washed, dried, and autoradiographed. Colonies that showed a strong hydridization signal with induced cDNA and a weak hybridization signal with normal cDNA were selected for further analysis. Plasmid DNA was isolated, cleaved with restriction endonuclease PstI and the cleavage fragments separated by electrophoresis in horizontal 1.5% agarose gels. Plasmids containing large ds-cDNA inserts were selected for further analysis.

Optionally, following the differential screening and the plasmid DNA isolation steps above, DNA clones which code for INF-$\gamma$ mRNA specifically may be identified by positive hybridization selection. DNA from a clone displaying a strong differential hybridization signal is immobilized on nitrocellulose filters as follows. Between 5 and 10 ug of DNA is adjusted to 0.2N NH$_4$OH and 2M NaCl in a small volume (<100 ul) and briefly heated to 100°C to totally denature the DNA. The DNA is immediately spotted onto a small piece of nitrocellulose filter paper. The filter is first air dried and then the DNA is covalently attached to the filter by heating at 80°C for two hours. The filter is incubated in a small volume of 70% (v/v) formamide, 0.3M NaCl, 10 mM Hepes (pH 7.5), 0.2% SDS and 5 mM EDTA for 5 min at 47°C. The hybridization between the specific cDNA clone and 10-20 μg of unlabeled IFN-γ. mRNA is carried out for three hours at 47°C in essentially the same buffer. The unreacted mRNA is then removed by washing five times at 47°C in a solution of 0.75 mM NaCl, 7.5 mM sodium citrate (pH 7.4) and 0.5% SDS. The mRNA complimentary to the cDNA is then eluted by two successive 10 min incubations at 47°C in 90% (v/v) formamide in 0.1% SDS, 10 mM Hepes (pH 7.5), 1 mM EDTA and 33 μg/ml of carrier tRNA 9to aid in precipitation of the specific mRNA). The RNA is precipitated at least two times with ethanol and then translated in an $\underline{in\ vitro}$ or $\underline{in\ vivo}$ translating system. The protein encoded for by the IFN-γ mRNA can be identified by immunoprecipitation antisera to IFN-γ.

To further confirm the identity of the candidate IFN-γ plasmids, a partial nucleotide sequence of the ds-cDNA insert are determined. DNA inserts are labeled at the 3'-end with $^{32}$P-cordecypin and then subjected to DNA sequence analysis.

Expression of IFN-γ

High-level expression of IFN-γ in bacterial cells, is obtained when the ds-cDNA is cloned in a generalized expression vector which contains the $\underline{E.\ coli}$ trp promoter-operator region. Additional expression vectors used include pBR322 vectors into which a synthetic ribosome binding site has been inserted into the β-lactamase gene.

High level of expression of IFN-γ in yeast is obtained when the ds-cDNA is inserted into an appropriate site in the yeast 2 μ circle to bring expression of IFN-γ directly under the control of the 2 μ promoter function. The IFN- γ ds-cDNA are also inserted into yeast DNA in such a fashion that expression of the IFN- γ gene comes under the control of a derepressible yeast enzyme such as acid phosphate gene, pho E. High levels of expression of interferon protein are obtained by inserting this entire construction into the free replicating yeast 2 μ circle.

Expression of IFN- γ in mammalian cells is obtained when the ds-cDNA insert is placed under the control of the SV40 early and late promoters. The recombinant DNA is used to transfect mammalian cells in order to obtain appropriate post-translational modification.

## EXAMPLE 9

This example presents the analysis of IFN-$\gamma$ after purification according to method described in the inventor's U.S. Application No. 437,660. Briefly, the purification was carried as follows: A solution of centrifuged crude immune interferon was adjusted to 500 mM with powdered Tris and the pH adjusted to 9.5 with 12N HCl. The adjusted crude interferon was loaded onto a Controlled Pore Glass Bead (CPG) column at a flow rate of '=500 ml/hr (=94 ml/cm$^2$/hr). The column was monitored at 2.0AU and the unbound material collected into a beaker. The CPG column was washed with a 500 mM Tris solution, pH 9.5, until the optical density (OD$_{280}$) was 0. The column was monitored at 2.0 AU and the unbound material collected. THe column is washed with phosphate buffered saline (PBS) until until OD$_{280}$ returns to baseline. (Flow rate 80 ml/hr overnight). The eluted material is collected into a second beaker. The CPG column was eluted with 2M ammonium sulfate at pH 9.0 at a flow rate of 24 ml/hr, and 10 ml fractions were collected. When second peak began to appear, the inlet of a Concanavalin A-Sepharose (Con A) column was connected into the outlet of the monitor and the outlet of the Con A column into the fraction collector. The CPG column eluted onto the Con A column at a flow rate of 24 ml/hr (12/cm$^2$/hr thru Con A). The column was monitored as above and 10 ml fractions collected until the OD$_{280}$ reached the baseline.

The CPG column was disconnected from the monitor and the monitor connected to the Con A column. The Con A column was washed with PBS until OD$_{280}$ returned to baseline. The flow rates and collections were monitored as above. The inlet of a Heparin-Sepharose (H/S) column, equilibrated in 20 mM phosphate buffer (PB),was connected to the outlet of the monitor and the outlet of the H/S to the fraction collector. The Con A column was eluted with the PBS sugar solution. The flow rate and collect were monitored as above until the $OD_{280}$ returned to baseline.

The Con A column was disconnected from the monitor and the H/S connected to the monitor. The H/S column was washed with PB until $OD_{280}$ returned to baseline. The column was then washed with a neutral pH buffer containing a physiological concentration of sodium chloride (PBS) until the $OD_{280}$ returned to baseline. The flow rate and collection were monitored as above. The H/S column was eluted with 2M NaCl in PBS, pH 7.2 until $OD_{280}$ returned to baseline. The flow rate monitored as above and 3 ml fractions were collected. The H/S peak fractions were selected by $OD_{280}$ then collected and pooled.

The pooled material was exhaustively dialyzed twice against 10 mM Tris-HCl, pH 9.5. This material was then loaded onto the carboxymethyl agarose (CM-A) column equilibrated in the same buffer. After the material was loaded, the column was washed with the same buffer until the $OD_{280}$ returned to baseline. The interferon was then eluted off the column in an ascending fashion with 20 mM tris-HCl, pH 9.5 containing 50 mM sodium chloride.

The pooled material was loaded onto the drained bed of the first of two gel filtration columns (26 X 94cm), containing AcA54 (900ml bed volume) equilibrated with 2M NaCl in PBS and hooked up in series, at 20 ml/hr by gravity (=80% bed height pressure head). After sample was loaded, the sides of column were washed with 2M NaCl in PBS, pH 7.2. The flow was stopped and the column topped off with PBS. The flow was then opened and the column run at 24 ml/hr in PBS pH 7.2 with 2M NaCl. The system was monitored at 0.5AU and 10 ml. fractions were collected. The results in Table I show the degree of purification and recovery of human immune interferon when purified by the sequential purification techniques described above.

TABLE I

| Step | Total Units | Total Protein (mg) | Specific Activity (units/mg) | Degree of Purification (fold) | Recovery per step (%) |
|------|-------------|--------------------|------------------------------|-------------------------------|------------------------|
| Crude | $3.2 \times 10^8$ | 384,000 | $8.3 \times 10^2$ | -- | -- |
| CPG<br>Con A $\}$ [a]<br>H/S | $3.0 \times 10^8$ | 57.6 | $5.2 \times 10^6$ | 6,265 | 93.7 |
| CM-BGA[b] | $9.8 \times 10^7$ | 3.4 | $2.9 \times 10^7$ | 34,939 | 32.6 |
| AcA 54[c] | | | | | |
| Overall | $1.0 \times 10^8$ | 1.69 | $5.9 \times 10^7$ | 71,084 | 102.0 |
| Frac. 34 | $9.2 \times 10^6$ | 0.86 | $1.0 \times 10^7$ | 12,048 | 9.4 |
| 35 | $5.7 \times 10^7$ | 0.23 | $2.5 \times 10^8$ | 301,204 | 58.1 |
| 36 | $2.9 \times 10^7$ | 0.31 | $9.3 \times 10^7$ | 112,048 | 29.6 |
| 37 | $9.2 \times 10^6$ | 0.29 | $3.1 \times 10^7$ | 37,349 | 9.4 |

[a]CPG - Controlled Pore Glass Bead Column
ConA - Concanavaline A-Sepharose Column
H/S - Heparin-Sepharose Column

[b]CM-BGA - Carboxymethyl-Agarose Column

[c]AcA54 - Gel Filtration Column

Optionally, after final elution, the IFN-γ is further contacted with phenyl-Sepharose for a period of time sufficient to accomplish equilibrated absorption of the IFN-γ to the phenyl-Sepharose and the eluting the absorbed IFN-γ with low salt buffers resulting in a further purification of the IFN-γ .

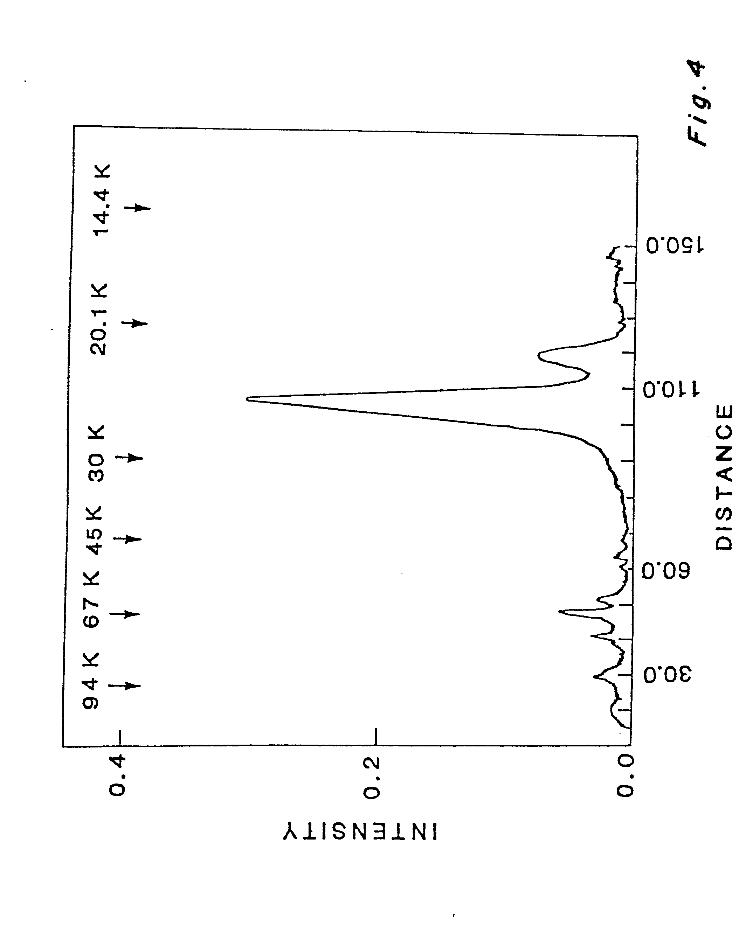

Based on SDS-PAGE separation procedures, the purified IFN-γ was resolved into one major band and several minor bands. Figure 4 shows a scan at 560nm of Coomassie Blue stained gels. By use of monoclonal antibodies directed against IFN α, β and γ it was determined that: (1) the major band which migrated at a rate corresponding to 26,000 ± 3,000 daltons and the minor bands which migrated at 22,000 ± 3,000 daltons were IFN-γ ; (2) the preparation is about 90-92% pure (i.e., substantially homogeneous); and (3) the specific activity of the preparation is approximately $2.8 \times 10^8$ Units/mg. The high molecular bands may also represent IFN-γ species however this has not be confimed. The bands corresponding to the 22K and 26K species were cut out and subjected to amino acid analysis in a Durham Amino Acid Analyzer. The results of the analysis are present in Table II.

## TABLE II

| Amino Acid | Nucleotide | 26K Species* | 22K Species* |
|------------|-----------|--------------|--------------|
| Asx | 20 | 20.4 | 20.3 |
| Thr | 5 | 5.5 | 6.2 |
| Ser | 11 | 12.0 | 11.9 |
| Glx | 19 | 20.4 | 21.0 |
| Pro | 2 | 3.1 | 2.2 |
| Gly | 5 | 7.1 | 7.4 |
| Ala | 8 | 9.8 | 9.8 |
| Cys | 2 | N.D. | N.D. |
| Val | 8 | 5.4 | 7.0 |
| Met | 4 | 2.4 | 1.4 |
| Ile | 7 | 6.2 | 5.7 |
| Leu | 10 | 13.2 | 12.9 |
| Tyr | 5 | 2.4 | 2.8 |
| Phe | 10 | 7.5 | 8.9 |
| His | 2 | 3.5 | 2.7 |
| Lys | 20 | 19.0 | 18.1 |
| Arg | 7 | 4.6 | 4.8 |
| Trp | 1 | N.D. | N.D. |

* The composition was computed from the known nucleotide sequence and adjusted for 143 residues.

To further characterize the two major IFN-$\gamma$ species, the 22,000 and 26,000 dalton species were individually subjected to CNBr degradation and the resulting polypeptides were partially sequenced in an automated protein sequentor. The partial sequences of the 22,000 and 26,000 dalton species are presented in Table III and are correlated with the published nucleotide sequence for the same region.

## TABLE III

| Position   | 81  | 82  | 83  | 84  | 85  | 86  | 121 | 122 | 123 | 124 | 125 | 126 |
|------------|-----|-----|-----|-----|-----|-----|-----|-----|-----|-----|-----|-----|
| Nucleotide | Asn | Val | Lys | Phe | Phe | Asn | Ala | Glu | Leu | Ser | Pro | Ala |
| 26 K       | Asn | Val | Lys | Phe | Phe | Asn | Ala | Glu | Leu | Ser | Pro | Ala |

| Position   | 121 | 122 | 123 | 124 | 125 | 126 | 127 | 128 | 129 | 130 | 131 |
|------------|-----|-----|-----|-----|-----|-----|-----|-----|-----|-----|-----|
| Nucleotide | Ala | Glu | Leu | Ser | Pro | Ala | Ala | Lys | Thr | Gly | Lys |
| 22 K       | Ala | X   | X   | Ser | Pro | Ala | Ala | Lys | Thr | Gly | Lys |

-47-

It should be noted that the 22,000 and 26,000 dalton species of IFN-$\gamma$ of Example 9 correspond to the 25,000 and 28,000 dalton species of Example 7, the differences being attributable to the method of SDS-PAGE analysis, which emphasize the need to take into consideration the method of gel analysis when interpreting the molecular weight parameters. In the case of Example 7, the SDS-PAGE procedure was conducted in cylindrical gels, with the molecular weight marker proteins being run concurrently but in separate cylindrical gels. In contrast the SDS-PAGE analysis of IFN-$\gamma$ in Example 9 was performed in a slab gel system with the molecular weight markers being run in parallel channels within the same gel. This latter system is known to provide a more accurate molecular weight estimate for IFN-$\gamma$. The variation between the compositional analyses disclosed in Examples 7 and 9 is due to the presence of residual $NH_4^+$ in the gels of the material analyzed in Example 7, whereas the gels of Example 9 were extensively dialyzed to remove the excess $NH_4^+$ prior to subjecting the sample for amino acid analysis. The excess $NH_4^+$ resulted from the $NH_4^+$ persulfate catalyst employed to form the gels. As is known, the analysis of basic amino acids is particularly susceptible to variation due to excess $NH_4^+$ in the gels. Although the results presented in Example 7 (in the presence of excess $NH_4^+$) and the results presented in Example 9 (in which the excess $NH_4^+$ has been removed) are both reproducible, it is likely that the data presented in Example 9 more accurately reflects the true amino acid composition of the IFN-$\gamma$. Further the absence of a species of IFN-$\gamma$ in this Example which corresponds to the 20,000 dalton species in Example 7, lends credence to the notion that the species identified as $\gamma_1$, and $\gamma_2$ in Example 7 represent two forms of IFN-$\gamma$ having substantially the same amino acid composition rather than different compositions, the compositioned differences observed between the two species likely being due to the presence of excess $NH_4^+$ during the analysis of same.

While the invention has been described in terms of preferred embodiments constituting the best mode known to the applicants at the time of this application, various changes may be made in the invention without departing from the scope thereof, which is defined by the following claims.

## What is Claimed is:

1. In a process for the production of IFN-$\gamma$ comprising incubating for a period of time at least sufficient for the production of IFN-$\gamma$, a viable cell suspension which includes leukocytes, an inducer for production of IFN-$\gamma$, and a modulator for said inducer selected from mezerein or 12,13-phorbol butyrate, said inducer and said modulator being present in the suspension in amounts sufficient to stimulate production of IFN-$\gamma$, separating the supermatatant from the cell suspension and purifying the IFN-$\gamma$ by

(a) adsorbing the IFN-$\gamma$ onto a column containing Controlled Pore Glass beads and eluting with ammonium sulfate;

(b) adsorbing the IFN-$\gamma$ containing eluate of step (a) onto a column containing Concanavalin A-Sepharose, lentil lectin-Sepharose or pea lectin-agarose and eluting with a buffer containing a sugar;

(c) adsorbing the IFN-$\gamma$ containing eluate of step (b) onto a column containing Heparin-Sepharose or Procian Red-agarose and eluting with a buffer containing a high salt content; and

(d) passing the IFN-$\gamma$ containing eluate of step (c) through a gel-filtration column equilibrated in high salt and recovering purified IFN-$\gamma$; characterized by dialyzing the partially purified interferon against a chemically compatible buffer and contacting the solution of dialyzed partially pure interferon with a column of cationic exchanger resin for a period of time sufficient to accomplish equilibrated adsorption of the interferon to the exchanger in the presence of a buffer at pH about 9.0 to 10.0, washing the resin having interferon adsorbed thereon from the soluble fraction with a buffered solution until the optical density of the eluate at 280 nm is about 0, eluting the adsorbed interferon with a desorbing buffer of about 10-30 mM tris-HCl at pH of about 9-10 containing 50 mM NaCl; the step being performed prior to step (d) and after steps (a), (b), (c).

2. The process according to Claim 1 wherein the cationic exchanger is a carboxymethyl, phospho or sulphopropyl group.

3. The process according to Claim 1 or 2 wherein the buffered solution is tris(hydroxymethyl) amino methane-HCl having a concentration of about 10 mM and a pH of about 9.2-9.8.

4. The process according to any of Claims 1 to 3 wherein the desorbing buffer of step (d) is tris(hydroxymethyl) amino methane-HCl at a concentration of about 20mM at a pH of about 9.5 and containing about 50 mM NaCl.

5. The process according to any of Claims 1 to 4 wherein the final elution IFN-$\gamma$ is further contacted with phenyl-Sepharose for a period of time sufficient to accomplish equilibrated adsorption of said IFN-$\gamma$ to the phenyl-Sepharose, and eluting the adsorbed IFN-$\gamma$ with low salt buffers.

6. A process according to any of Claims 1 to 5 which comprises:

(a)(i) contacting said IFN-$\gamma$ with Controlled Pore Glass beads for a period of time sufficient to adsorb said IFN-$\gamma$ onto said beads in the presence of a neutral pH buffer;

(ii) washing said beads with a chemically compatible buffer until the optical density of the eluant at 280 nm is about 0;

(iii) washing said beads with a neutral pH buffer solution until the optical density of the eluate at 280 nm is about 0; and,

(iv) eluting IFN-$\gamma$ from said beads with an ammonium sulfate solution;

(b)(i) contacting said eluate of step (a)(iv) with an adsorbent selected from the group consisting of Concanavalin A-Sepharose, lentil lectin-Sepharose and pea lectin-agarose for a period of time sufficient to adsorb said IFN-$\gamma$ onto said adsorbent in the present of a neutral pH buffer;

(ii) washing said adsorbent with ammonium sulfate until the optical density of the eluant with 280 nm is about 0;

(iii) washing said adsorbent with a neutral pH buffer until the optical density of the eluate at 280 nm is about 0; and,

(iv) eluting IFN-$\gamma$ from said adsorbent with a buffer containing a sugar of the group consisting of alpha-methyl D-mannoside and 1-methyl D-glucoside;

(c)(i) contacting said eluate of step (b)(iv) with an adsorbent selected from the group consisting of Heparin-Sepharose and Procian Red-agarose for a period of time sufficient to adsorb said IFN-$\gamma$ onto said adsorbent in the presence of a neutral pH buffer;

(ii) washing said adsorbent with a neutral pH buffer containing a sugar selected from the group consisting of alpha-methyl D-mannoside and 1-methyl D-glycoside until the optical density of the eluate at 280 nm is about 0;

(iii) washing said adsorbent with a neutral pH buffer until the optical density of the eluate is about 0; and,

(iv)   washing said adsorbent with a neutral pH buffer containing a physiological concentration of NaCl until the optical density of the eluate is about 0;

(v)   eluting the IFN-$\gamma$ from said adsorbent with a neutral pH buffer containing a high concentration of salt; and,

(d)(i)   passing said eluate of step (c)(iv) through a gel-filtration column equilibrated in a neutral pH buffer containing a high concentration of salt; and,

(ii)   eluting IFN-$\gamma$ from said column with a neutral pH buffer.

7.   The species of homogeneous human IFN-$\gamma$ corresponding to a slab SDS-PAGE molecular weight band of about 22,000 ± 3,000 daltons.

8.   The species of said homogeneous human IFN-$\gamma$ corresponding to a slab SDS-PAGE molecular weight band of about   26,000 ± 3,000 daltons.

9.   The species according to Claim 7 wherein said IFN-$\gamma$ is characterized by the partial amino acid sequence Asn-Val-Lyl-Phe-Phe-Asn-Ala-Glu-Leu-Ser-Pro-Ala-.

10.   The species according to Claim 8 wherein said IFN-$\gamma$ is characterized by the partial amino acid sequence Ala-X-X-Ser-Pro-Ala-Ala-Lys-Thr-Gly-Lys.

11.   IFN-$\gamma$ when prepared according to any of Claims 1 to 6.

BLOOD → CENTRIFUGE → BUFFY COATS → SUSPEND IN CULTURE MEDIUM CONTAINING ANTIBIOTICS → CONDITION → ADD PROTEIN SOURCE → INCUBATE STATIONARY / INCUBATE STIR → CENTRIFUGE

PLASMA

REMOVE RED BLOOD CELLS

LEUKO-CYTES

ADMIX MODULATOR AND INDUCER

SUPER-NATANT (CRUDE IFN-γ)

PURIFY

HOMO-GENEOUS IFN-γ

SPECIES 1    SPECIES 2

CELLS CONTAINING m-RNA

FIG. 1 SH. 1
FIG. 1 SH. 2

*Fig. 1*

FRACTIONS CONTAINING m-RNA WITH HIGH IFN-γ ACTIVITY ← SAMPLES FROM FRACTIONS ARE ASSAYED FOR ABILITY TO PRODUCE IFN-γ IN FROG OOCYTES ← SIZE FRACTION-ATE ELUATE ← PASS THROUGH POLY(U) COLUMN AND ELUTE m-RNA ← EXTRACT LARGE DNA STRANDS AND PROTEIN ← LYSE CELLS

TO FIG. 1 SHEET 2

*Fig. 1*
(Sheet 1)

1/4

0137691

Fig. I
(Sheet 2)

Fig. 3

MEZEREIN

DOSE RESPONSE OF MEZEREIN (ng./ml.)
IN .5 ug./ml. OF A-23187.

Fig. 2

A-23187

DOSE RESPONSE OF A-23187 (ug./ml.)
IN 70 ng./ml. OF MEZEREIN.

INTERFERON (x10³ UNITS/ml.)

Fig.4

0137691

4 / 4

# EUROPEAN SEARCH REPORT

European Patent Office

| DOCUMENTS CONSIDERED TO BE RELEVANT | | EP 84305859.5 |

| Category | Citation of document with indication, where appropriate, of relevant passages | Relevant to claim | CLASSIFICATION OF THE APPLICATION (Int. Cl.4) |
|---|---|---|---|
| A,D | US - A - 4 382 027 (BRAUDE)<br>* Claims 1,15 * | 1,5,6 | C 12 P 21/02<br>C 07 K 13/00<br>//C 12 N 15/00 |
| A | EP - A2 - 0 077 670 (GENENTECH.)<br>* Page 43, line 9 - page 44, line 11; fig. 5 * | 1,2,6-11 | |
| A,D | US - A - 4 376 821 (BRAUDE)<br>* Claim 1; column 4, lines 26-45 * | 1 | |
| A | WO - A1 - 81/03 498 (NEW YORK UNI-VERSITY) | | |
| | | | TECHNICAL FIELDS SEARCHED (Int. Cl.4)<br><br>C 12 P<br>C 07 K<br>C 12 N<br>A 61 K |

| The present search report has been drawn up for all claims | | |
|---|---|---|
| Place of search | Date of completion of the search | Examiner |
| VIENNA | 06-12-1984 | FARNIOK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82